# EUROPEAN PATENT SPECIFICATION

(11) **EP 1 699 351 B1**
(45) Date of publication and mention of the grant of the patent: **04.05.2011**
(21) Application number: 04815765.5
(22) Date of filing: 22.12.2004
(51) Int. Cl.: A61B 5/00, G01N 21/65

(54) **PROCESS FOR DETERMINATION OF CELL VIABILITY**
VERFAHREN ZUR BESTIMMUNG DER LEBENSFÄHIGKEIT VON ZELLEN
PROCEDE PERMETTANT DE DETERMINER LA VIABILITE DES CELLULES

(30) Priority: 22.12.2003 US 531848 P
(43) Date of publication of application: 13.09.2006
(73) Proprietor: Lightouch Medical, Inc., Bryn Athyn, PA 19009 (US)
(72) Inventor: CHAIKEN, Joseph, Fayetteville, NY 13066 (US); DRACKER, Robert, Liverpool, NY 13090 (US); HAGRMAN, Pamela, J., Syracuse, NY 13215 (US); HAGRMAN, Douglas, Syracuse, NY 13215 (US)
(74) Representative: Campbell, Patrick John Henry
(86) International application number: PCT/US2004/043759
(87) International publication number: WO 2005/063119

(56) References cited:
- EP-A- 0 818 674
- US-A- 6 149 868
- US-A1- 2002 132 371
- LI T ET AL: "RAMAN DYNAMIC PROBE OF HYDROGEN EXCHANGE IN BEAN POD MOTTLE VIRUS: BASE-SPECIFIC RETARDATION OF EXCHANGE IN PACKAGED SSRNA" BIOPHYSICAL JOURNAL, NEW YORK, US, US, vol. 65, no. 5, November 1993 (1993-11), pages 1963-1972, XP002056619 ISSN: 0006-3495
- UJJ L ET AL: "PICOSECOND RESONANCE COHERENT ANTI-STOKES RAMAN SPECTROSCOPY OF BACTERIORHODOPSIN: SPECTRA AND QUANTITATIVE THIRD-ORDER SUSCEPTIBILITY ANALYSIS OF THE LIGHT-ADAPTED BR-570" CHEMICAL PHYSICS, AMSTERDAM, NL, vol. 182, no. 2/3, 1 May 1994 (1994-05-01), pages 291-311, XP002056618

## Description

### TECHNICAL FIELD OF THE INVENTION

The invention relates generally to a process for the determination of cell viability.

### BACKGROUND OF THE INVENTION

Various applications including the use of tissue and cell culture to produce materials for treatment of various health problems creates the necessity to quantitatively assess the viability of cells and tissues at all stages of growth and propagation. Currently this is accomplished using dye exclusion and other approaches that are messy, time-consuming, expensive, difficult to automate and perform with rapid throughput, not easy to quantitate and, in the final analysis, not very precise and accurate. The process of dye exclusion can also consume or modify some of the cells and, in the case of stem cells, the loss of the cells themselves is an extreme disadvantage. Thus, there exists a need for an improved process to determine cell and tissue viability.

EP 0 818 674 teaches a method for determining presence of cells.

### SUMMARY OF THE INVENTION

The invention is defined in claim 1 and provides a method to determine the viability of cells by measuring the absolute and relative rate of metabolic activity and/or integrity of the cell membrane through the use of vibrational spectroscopy.

In one embodiment, the method comprises obtaining a container loaded with deuterated materials, introducing cells into the container whereby the cells are in contact with the deuterated materials, and obtaining vibrational spectra emitted by the cells. The vibrational spectra emitted by the cells are indicative of metabolism, thereby providing an indication of viability of the cells, such that greater metabolic activity is indicative of greater viability.

The vibrational spectra can be Raman spectra, infrared and/or near infrared spectra. The deuterated material can be, for example, selected from the group consisting of α-D-glucose; 6,6- dideutero-α-D-glucose; D₂O; 3-O-methylglucose; 6,6- dideutero-α-D-3-O-trideuteromethylglucose; 6,6- dideutero-α-D-3-O-methylglucose; 6,6- dideutero -α-D-2-O-methylglucose; and deuterated amino acids.

In some embodiments, the method further comprises normalizing the Raman spectra obtained by comparing the Raman spectra obtained at a target wavenumber to the Raman spectra obtained at a reference wavenumber. Representative target wavenumbers include 960 cm⁻¹, 1270 cm⁻¹ and 2400-2600 cm⁻¹. Typical reference wavenumbers include the amide I Raman feature (1600-1700 cm⁻¹), the CH₂ Raman feature (1450 cm⁻¹), the amide III Raman feature (1200-1350 cm⁻¹), the CH stretching region (2900-3000 cm⁻¹), and the integral of all the Raman features (300-1850 cm⁻¹). In some embodiments, the Raman spectra are normalized by comparing the Raman spectra obtained at a target wavenumber to the fluorescence generated by a Raman excitation source, such as the H₂O fluorescence (980 nm).

In another embodiment, the method of determining cell viability comprises obtaining a container loaded with deuterated materials, introducing cells into the container whereby the cells are in contact with the deuterated materials, obtaining vibrational spectra emitted by the cells, placing an aliquot of the cells into a medium free of deuterated materials, obtaining vibrational spectra emitted by the cells in the non-deuterated medium, and determining the rate of decrease of emitted vibrational spectra. The vibrational spectra are indicative of metabolism, thereby providing an indication of viability of the cells, such that a faster rate of decrease of emitted vibrational spectra is indicative of greater viability.

### BRIEF DESCRIPTION OF THE FIGURES

Figure 1 shows spectral counts as a function of Raman shift (in cm⁻¹) measured from human leukocytes grown in culture and exposed to phosphate buffered saline for 30-90 minutes. These spectra serve as a control for comparison to those shown in Figure 2.
Figure 2 shows spectral counts as a function of Raman shift (in cm⁻¹) measured from human leukocytes grown in culture and exposed to phosphate buffered saline containing D₂O instead of H₂O for 30-90 minutes.

### DETAILED DESCRIPTION

One of the most basic indicators of viability is the metabolism of glucose and the active maintenance of membrane integrity. A healthy cell keeps certain materials on the inside and other materials on the outside of its membranes or cell wall (in the case of plant. cells). While it is possible to synthesize vesicles and micelles and other objects that exclude, to a greater or lesser extent, certain materials from their inner volume, it is not possible, using such entities, to mimic the full range of kinetics and thermodynamic behavior of living, i.e. metabolizing, cells or tissues. Having the ability to noninvasively monitor the consumption of nutrients and/or production of metabolites or waste allows a temporally continuous, direct measure of cell viability from the moment of harvest from the primary source, e.g. stem cells from umbilical cord blood, to the final growth stages of some particular daughter culture. A highly optimized and novel approach to accomplishing this type of measurement system is provided by this invention.

Minimization of sample preparation is a worthy goal because variability and inefficiency at that stage of analysis affects accuracy, precision, throughput and the cost structure of the overall process. Given the turbidity of cultures at all stages of handling, having a method that is tolerant of turbidity is desirable. This feature, and the inherent need to work in what is always an essentially aqueous environment, discourages analytical approaches based on absorption spectroscopy. UV-visible spectroscopy has little specificity (without the addition of what are essentially invasive fluorophores) and mid-infrared spectroscopy has solvent absorption limitations. Absorption in the near IR is feasible for some analytes, but the isolation and unambiguous association of spectral features with specific analytes has proven to be challenging at millimolar concentrations for tissue-like samples.

### Definitions

All scientific and technical terms used in this application have meanings commonly used in the art unless otherwise specified. As used in this application, the following words or phrases have the meanings specified.

As used herein, "vibrational spectroscopy" refers to spectroscopic techniques known in the art to be based on vibrational features, including Raman, resonance Raman, infrared (IR) and near infrared (NIR) spectroscopy.

As used herein, "Raman spectra associated with" a given component refers to those emitted Raman spectra that one skilled in the art would attribute to that component. One can determine which Raman spectra are attributable to a given component by irradiating that component in a relatively pure form, and collecting and analyzing the Raman spectra emitted by the component in the relative absence of other components. Those skilled in the art are aware of available libraries that catalog known Raman spectra.

As used herein, "metabolism" refers to the physical and chemical processes occurring within a living cell that are necessary for the maintenance of life, including anabolism and catabolism, and the processes by which a particular substance is handled (as by assimilation and incorporation or by detoxification and excretion).

As used herein, unless context clearly indicates otherwise, "a" means at least one, and can include a plurality.

### Determination of Cell Viability

Viability can be assessed using spectroscopy in many ways. One example to is to use standard chemometric techniques to integrate the observed Raman activity in and around the vicinity of 2200-2400 cm⁻¹ Raman shift (Stokes) (the C-D stretching modes) and the activity in and around 1600-1700 cm⁻¹ (amide I). The ratio of these two integrated signals is zero before the cells have absorbed any of the "deuterated viability agent" and increases when the cells are incubated with the deuterated agent. The ratio has a different time course depending on the specific nature of the agent used and the viability of the cells. Utilizing glucose as the agent, for example, a population of more viable cells takes up the agent faster than a population of less viable cells and slower than a population of cancerous cells.

The invention provides a method to determine the viability of cells by measuring the absolute and relative rate of metabolic processes. In one embodiment, the method measures indicia of glucose uptake, clearing and/or turnover into various analytes, e.g. lactate, based on using α-D-glucose, 6,6- dideutero-α-D-glucose, D₂O, 3-O-methylglucose, 6,6- dideutero-α-D-3-O-trideuteromethylglucose, 6,6- dideutero-α-D-3-O-methylglucose, -α-D-2-O-methylglucose, and other deuterated forms of constituents of nutrient broths and agars associated with the particular cell/tissue types desired. In another embodiment, the method measures indicia of protein metabolism, such as by use of deuterated amino acids.

The method typically comprises contacting the cells of interest with a deuterated agent and probing vibrational spectra emitted by the cells. In one embodiment, the method comprises obtaining a container loaded with deuterated materials, introducing cells into the container whereby the cells are in contact with the deuterated materials, and obtaining vibrational spectra emitted by the cells. The vibrational spectra emitted by the cells are indicative of metabolism, thereby providing an indication of viability of the cells, such that greater metabolic activity is indicative of greater viability. The vibrational spectra can be Raman spectra (including resonance Raman), infrared and/or near infrared spectra. In some embodiments, the method further comprises subsequently placing the cells in an environment free of the deuterated materials. The rate of clearance of the deuterated material is then monitored to detect viability of the cells. Healthy, viable cells will clear the deuterated material more quickly than nonviable cells.

### Normalization

The method of the invention can be used for making comparisons between measurements and samples through the use of a "normalizer". In general, any process is more useful with a means for, within a particular sample of cells, controlling or correcting data for the number of cells being probed. A process that relies solely on measuring the intensity of a deuterium related spectral feature by itself is susceptible to errors induced by variation in sample volume, purity and other artifacts of sample preparation and handling. As one example, if at some point the sample of cells is pre-concentrated by centrifugation before spectroscopic probing, then the amount of spectroscopic signal obtained, e.g. Raman or fluorescence or absorption in some spectral region, will depend on how much of the original mother liquor is present or how dispersed the "plug" becomes during sample manipulation/transportation. The amount of the volume of the "plug" that is probed will also depend on the laser or other light source power in a nonlinear and generally complicated manner because there is always a particular geometry of the light delivery and collection optical systems. It is therefore generally useful to use some internal spectroscopic or possibly physical optical signal, i.e. Rayleigh scattered light, that is obtained simultaneously with the deuterated signal, that is indicative of the number of cells being probed or the volume being probed in cases where the density of cells can be independently ascertained.

To cite a few examples of possible normalizers, we note that all living cells contain protein, carbohydrate and lipid in relatively large quantities. Since spontaneous Raman scattering is generally speaking a parts per thousands analytic technique, or equivalently, only capable of providing quantitation down to millimolar concentrations, the choice of normalizer based on a Raman signal, as in the first example given above, is limited to materials that occur naturally in cells in appropriate concentrations. Normalization can be achieved by determining a ratio between spectral features at a target wavenumber and spectral features at a reference wavenumber. The area under a peak, in spectral counts at or spanning a range of the target wavenumber(s) is divided by the area under a peak in spectral counts at or spanning a range of the reference wavenumber(s). A target or reference wavenumber refers to the spectral feature associated with a particular attribute, such as a C-D bond (at 2400-2600 cm⁻¹).

In the case of vibrational spectroscopies, some spectroscopic features are more appropriate than others for the purpose of volume/cell number normalization. Although the concentration of a given protein may only be micromolar or below, each protein molecule contains thousands to tens of thousands of amide linkages. Each of these bonds produces a spectroscopic signal at nearly the same Raman shift and so the "piling up" of the signal makes the various amide related features, particularly amide I and amide III, strong and often measurable with high signal to noise ratio. Thus the example given above suggested using the so-called amide I signal as a normalizer. Clearly the amide III and other types of amide signals may also be appropriate depending on the specific circumstances of the system in question, e.g. the composition of the nutrient medium and the ability to wash away potentially interfering substances before normalization.

In some cases the normalizer will involve using the CH₂ deformation Raman feature at and around 1450 cm⁻¹. This feature will often be useful because it is a major constituent of phospholipid membranes that are an integral part of any animal cell. Each phospholipid molecule contains around 14-18 of these structural features and so the accumulation of these features allows a convenient and general method for normalization. Note that some nutrient media contain much more protein constituents, i.e. amino acids, than lipid and, in these cases, the adequacy of washing before spectroscopic cell viability assessment is less critical. In the same vein, assessment of plant cell viability will necessarily involve cellulose related features because cell walls are composed of cellulose.

The use of a normalizer is most important when the spectroscopic signals in question are obtained in two separate steps. That is, if the undeuterated spectroscopic signal is obtained using one device and the deuterated signal is obtained using another device, for example because the signals occur in different spectroscopic regions, then a normalizer is appropriate. If the two signals are obtained simultaneously, then a normalize as described is useful when comparing the results of two separate assessments. But, if the two signals are simultaneously obtained on the same device, then a normalize is not necessary. Nevertheless, the use of a normalizer may still improve the overall precision of the process.

In some embodiments, the 980 nm fluorescence of water is used as a normalizer in concert with Raman excited by a laser having wavelength not shorter than 785 and possibly as long as 980 nm. Choosing the laser wavelength in this range assures that there will be some water fluorescence that can be used to assess water content. The water content can be used as a volume normalizer, i.e. if the cell concentration is known, then a measure of the volume of water in a specific sample can be used as a measure of the number of cells being probed.

In one embodiment of the method, one first incubates the cells in question in an appropriately deuterated medium before removing cells in aliquots to incubate in nondeuterated medium. In this case, the same ratio is measured but now it decreases at a different rate for less viable cells than for more viable cells. In the case of using another analyte in conjunction with glucose (e.g. lactate), there will be more than one Raman feature in the 2200-2500 cm⁻¹ range. A ratio is formed involving each of these signals and either the amide I (1600-1700 cm⁻¹) or the amide III (1200-1350 cm⁻¹) as the denominator. The percentage decrease of one feature in the 2200-2500 cm⁻¹ range should be matched by an equal percentage increase in the other consistent with the stoichiometry of the overall process.

The amide I Raman feature (1600-1700 cm⁻¹) in the spectra of the cells and tissues and surrounding medium can be used as a normalizer (fluorescence) for the deuterium specific Raman features to obtain a "specific activity" of the deuterium. The specific activity can be calculated upon probing cell volume, tissue volume or volume of extracellular medium. This quantity will be useful in obtaining optimal quantitative data/analysis for use as a kinetic probe of cell-tissue viability.

Fluorescence generated by Raman excitation source can be used as a measure of cell volume, tissue volume or volume of extra-cellular medium being probed by Raman features. See, e.g., U.S. Patent No. 6,044,285. In general, porphyrin and long chain terpenoid and similarly derived substances, such as cytochrome, hemoglobin, chlorophyll and the carotenes, respectively, are known to fluoresce in the NIR, thus providing an opportunity to normalize for cell volume without adding exogenous substances, such as fluorophores.

The amide III Raman feature (1200-1350 cm⁻¹) in the spectra of the cells and tissues and surrounding medium can be used as a normalizer for the deuterium specific Raman features to obtain a "specific activity" of the deuterium. The specific activity can be calculated upon probing cell volume, tissue volume or volume of extra-cellular medium. This quantity will be useful in obtaining the best quantitative data/analysis for use as a kinetic probe of cell-tissue viability.

The H₂O fluorescence (980 nm) generated by Raman or a separate excitation source is useful as a measure of cell volume, tissue volume or volume of extra-cellular medium being probed by Raman features.

The use of the integral of all the Raman features (300-1850 cm⁻¹) in the spectra of the cells and tissues and surrounding medium can serve as a normalizer for the deuterium specific Raman features to obtain a "specific activity" of the deuterium. The specific activity can be calculated upon probing cell volume, tissue volume or volume of extracellular medium. This quantity will be useful in obtaining optimal quantitative data/analysis for use as a kinetic probe of cell-tissue viability.

### Kits

The invention provides kits for carrying out the methods of the invention. In one embodiment, the invention provides a plate with wells, depression(s) or compartment(s) into which a sample of cell or tissue culture can be placed and the spectroscopic process of this invention can be applied. In some embodiments, the plate is preloaded with appropriate deuterated materials to allow multiple kinetic measurements to be made to determine cell/tissue viability. The combination of this process and associated device with the aspects of a plate reader needed to provide cell count information allow the invention to provide absolute calibration to the device. The invention also provides preloaded centrifuge tubes that can be loaded with the cells, processed and then spectroscopically probed in the spun-down tube full of cells. Typically, the centrifuge tubes comprise glass or plastic. In a typical embodiment, the last step in the processing is a spin down step, e.g. following a series of washes.

### EXAMPLES

The following examples are presented to illustrate the present invention and to assist one of ordinary skill in making and using the same. The examples are not intended in any way to otherwise limit the scope of the invention.

### Example 1: Raman Spectra of Living Cells

This Example demonstrates that the spectroscopic analysis methods can be used to characterize viability of living cells. In this example, phosphate buffered saline (PBS) is used as a control to show that contacting cells with an isosmotic solution containing H₂O (rather than D₂O) does not alter spectroscopic features.

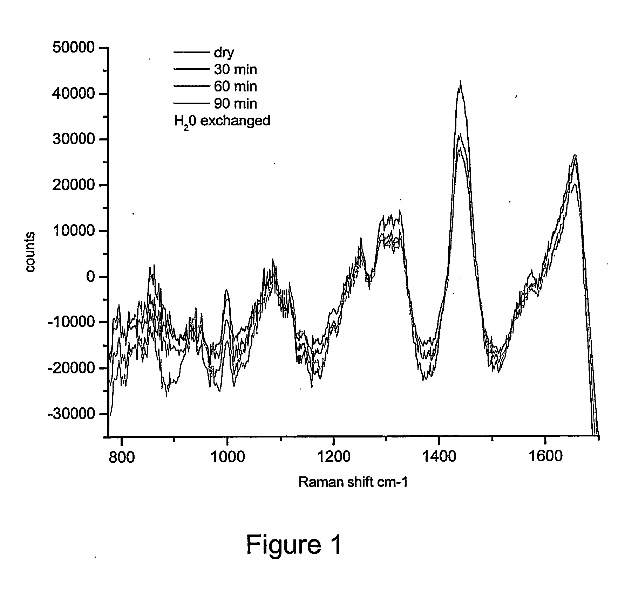

Human leukocytes (HL60-leukemia) were grown by standard tissue culture techniques. An aliquot of the cells was taken from the main culture and exposed to H₂O in phosphate buffered saline (PBS). Raman spectra of the cells were taken at progressively later times to show the effect of the water in the saline solution on the cells. There is clearly very little effect of adding water, as shown in Figure 1. The largest change observed involved going from the "dry" cells to the water added state. Note, however, that the cells were not actually dry. They had been spun down and there was just a small amount of residual water inside but not on the top of the "plug". The change in the reflection loss at the top of the cell plug, which varied when the PBS was added, caused the relatively large change in going from "dry" to "wet".

### Example 2: Raman Spectra of Living Cells Using Deuterated Water

This Example demonstrates that the spectroscopic analysis methods can be used to characterize the membrane function of living cells using deuterated agents. Viable cells, with intact membranes, will exhibit features associated with D₂O replacement of H₂O over time. In contrast, nonviable cells will exhibit immediate D₂O replacement. Accordingly, the time course of D₂O exchange can be used as a measure of cell viability.

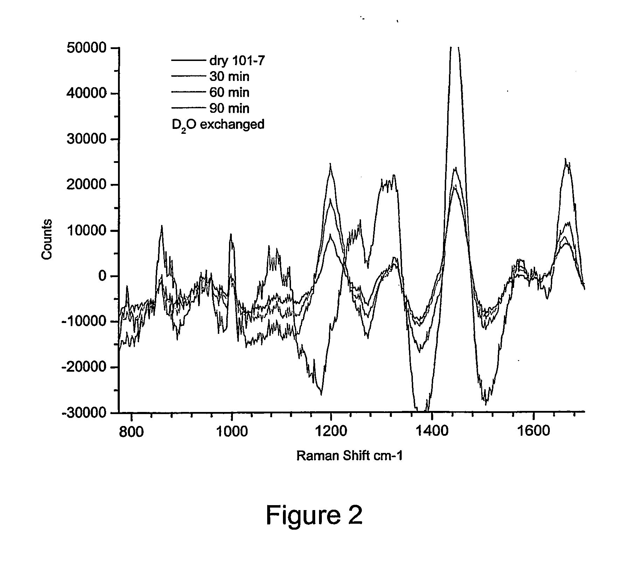

Figure 2 shows Raman spectra of human leukocytes that were grown by standard tissue culture techniques. The spectra were cut off at a slightly different Raman shift. The right-most peak looks rounded, but the spectra of both "dry" samples (used in Examples 1 and 2) were essentially identical. An aliquot of the cells was taken from the main culture and exposed to D₂O in phosphate buffered saline (PBS). Raman spectra of the cells were taken at progressively later times to show the effect of the deuterated water in the saline solution on the cells. There is clearly an effect. Note that the large change observed going from the "dry" cells ("dry" has same meaning as in Example 1) is the same as above, i.e. the change in the reflection loss at the top of the cell plug varied when either the deuterated or protonated PBS was added.

The large change near 1270 and 960 wavenumbers and other places as well is due to exchange of protons at the amide linkages. As exposure to the D₂O extends from 30 minutes to 60 minutes and then 90 minutes, significant changes are observed as D₂O is exchanged for H₂O. The rate of this exchange depends on the viability of the cells, as cells whose membranes have lost integrity will exhibit immediate changes on the order of changes that would take 90 minutes to occur in healthy, viable cells. The spectra in Figure 2 clearly show that deuterated agents can be employed in concert with live cells to probe their membrane function.

In order to compare the two sets of spectra quantitatively, normalization is used. For example, one could use the ratio of 1270 cm⁻¹ to 1450 cm⁻¹ in each set to compare to each other without having to use the 960 cm⁻¹ feature. Note also that these spectra do not cover a broad enough range to allow monitoring at 2400-2600 cm⁻¹. Accordingly, one could not simultaneously look at CD bonds and get the spectra shown below unless normalization of some kind is employed. In this case either the 1450 (CH₂ deformation) or the 1670 cm⁻¹ (amide I) could be used for normalization. Note most of the entire feature from 1200 to 1350 cm⁻¹ is called amide III. For both sets of spectra, the fluorescence has been subtracted off using a standard technique(101-7 as described in US 6,044,285), resulting in negative counts in some regions. We have produced the same demonstration using Jurket cells, another standard cell line often used in tissue culture.

Those skilled in the art will appreciate that the conceptions and specific embodiments disclosed in the foregoing description may be readily utilized as a basis for modifying or designing other embodiments for carrying out the same purposes of the present invention.

## Claims

1. A method of determining viability of living cells, comprising:
(a) obtaining a container loaded with deuterated materials;
(b) introducing living cells into the container whereby the living cells are in contact with the deuterated materials;
(c) obtaining vibrational spectra emitted by the living cells;
wherein the vibrational spectra emitted by the living cells are indicative of metabolism, thereby providing an indication of viability of the cells, such that greater metabolic activity is indicative of greater viability.

2. The method of claim 1, wherein the vibrational spectra are Raman spectra.

3. The method of claim 1, wherein the vibrational spectra are infrared or near infrared spectra.

4. The method of claim 1, wherein the deuterated material is selected from the group consisting of α-D-glucose; 6,6- dideutero-α-D-glucose; D₂O; 3-O-methylglucose; 6,6- dideutero-α-D-3-O-trideuteromethylglucose; 6,6- dideutero-α-D-3-O-methylglucose; and 6,6- dideutero -α-D-2-O-methylglucose.

5. The method of claim 1, wherein the deuterated material comprises a deuterated amino acid.

6. The method of claim 2, further comprising normalizing the Raman spectra obtained by comparing the Raman spectra obtained at a target wavenumber to the Raman spectra obtained at a reference wavenumber.

7. The method of claim 6, wherein the target wavenumber is 960 cm⁻¹, 1270 cm⁻¹ or 2400-2600 cm⁻¹.

8. The method of claim 6, wherein the reference wavenumber is the amide I Raman feature (1600-1700 cm⁻¹).

9. The method of claim 6, wherein the reference wavenumber is the CH₂ Raman feature (1450 cm⁻¹).

10. The method of claim 6, wherein the reference wavenumber is the CH stretch Raman feature (2900-3000 cm⁻¹).

11. The method of claim 6, wherein the reference wavenumber is the amide III Raman feature (1200-1350 cm⁻¹).

12. The method of claim 6, wherein the reference wavenumber is the integral of all the Raman features (300-1850 cm⁻¹).

13. The method of claim 2, further comprising normalizing the Raman spectra obtained by comparing the Raman spectra obtained at a target wavenumber to the fluorescence generated by a Raman excitation source.

14. The method of claim 13, wherein the fluorescence is the H₂O fluorescence (980 nm).

15. The method of claim 1, wherein the container is a centrifuge tube.

16. The method of claim 1, further comprising:
(a) placing an aliquot of the cells into a medium free of deuterated materials;
(b) obtaining vibrational spectra emitted by the cells in the non-deuterated medium;
(c) determining the rate of decrease of emitted vibrational spectra;
wherein a faster rate of decrease of emitted vibrational spectra is indicative of greater viability.

17. The method of claim 1, wherein a time course of D₂O exchange is used as a measure of cell viability.

## Patentansprüche

1. Verfahren zur Bestimmung der Lebensfähigkeit von lebenden Zellen, umfassend:
(a) Erhalten eines mit deuterierten Materialien beladenen Behälters;
(b) Einbringen von lebenden Zellen in den Behälter, wodurch die lebenden Zellen mit den deuterierten Materialien in Kontakt sind;
(c) Erhalten von Vibrationsspektren, die von den lebenden Zellen emittiert werden;
wobei die von den lebenden Zellen emittierten Vibrationsspektren ein Hinweis auf den Metabolismus sind, wodurch ein Lebensfähigkeitszeichen für die Zellen bereitgestellt wird, derart, dass eine größere Stoffwechselaktivität ein Hinweis auf eine größere Lebensfähigkeit ist.

2. Verfahren nach Anspruch 1, wobei die Vibrationsspektren Raman-Spektren sind.

3. Verfahren nach Anspruch 1, wobei die Vibrationsspektren Infrarot- oder nahe Infrarotspektren sind.

4. Verfahren nach Anspruch 1, wobei das deuterierte Material aus der Gruppe ausgewählt ist bestehend aus α-D-Glucose, 6,6-Dideutero-α-D-glucose; D₂O; 3-O-Methylglucose; 6,6-Dideutero-α-D-3-O-trideuteromethylglucose; 6,6-Dideutero-α-D-3-O-methylglucose; und 6,6-Dideutero-α-D-2-O-methylglucose.

5. Verfahren nach Anspruch 1, wobei das deuterierte Material eine deuterierte Aminosäure umfasst.

6. Verfahren nach Anspruch 2, weiterhin umfassend das Normieren der Raman-Spektren, die erhalten wurden durch Vergleichen der Raman-Spektren, die bei einer Ziel-Wellenzahl erhalten wurden, mit den Raman-Spektren, die bei einer Referenz-Wellenzahl erhalten wurden.

7. Verfahren nach Anspruch 6, wobei die Ziel-Wellenzahl 960 cm⁻¹, 1270 cm⁻¹ oder 2400-2600 cm⁻¹ ist.

8. Verfahren nach Anspruch 6, wobei die Referenz-Wellenzahl das Amid I-Raman-Charakteristikum (1600-1700 cm⁻¹) ist.

9. Verfahren nach Anspruch 6, wobei die Referenz-Wellenzahl das CH₂-Raman-Charakteristikum (1450 cm⁻¹) ist.

10. Verfahren nach Anspruch 6, wobei die Referenz-Wellenzahl das CH-Streck-Raman-Charakteristikum (2900-3000 cm⁻¹) ist.

11. Verfahren nach Anspruch 6, wobei die Referenz-Wellenzahl das Amid III-Raman-Charakteristikum (1200-1350 cm⁻¹) ist.

12. Verfahren nach Anspruch 6, wobei die Referenz-Wellenzahl das Intergral von sämtlichen Raman-Charakteristika (300-1850 cm⁻¹) ist.

13. Verfahren nach Anspruch 2, weiterhin umfassend das Normieren der Raman-Spektren, die erhalten wurden durch Vergleichen der Raman-Spektren, die bei einer Ziel-Wellenzahl erhalten wurden, mit der von einer Raman-Anregungsquelle erzeugten Fluoreszenz.

14. Verfahren nach Anspruch 13, wobei die Fluoreszenz die H₂O-Floureszenz (980 nm) ist.

15. Verfahren nach Anspruch 1, wobei der Behälter ein Zentrifugenröhrchen ist.

16. Verfahren nach Anspruch 1, weiterhin umfassend:
(a) Platzieren eines Aliquots der Zellen in einem von deuterierten Materialien freien Medium;
(b) Erhalten von durch die Zelle emittierten Vibrationsspektren in dem nicht deuterierten Medium;
(c) Bestimmen der Abnahmegeschwindigkeit der emittierten Vibrationsspektren;
wobei eine schnellere Abnahmegeschwindigkeit der emittierten Spektren ein Hinweis auf größere Lebensfähigkeit ist.

17. Verfahren nach Anspruch 1, wobei ein Zeitverlauf des D₂O-Austausches als ein Maß für die Zell-Lebensfähigkeit verwendet wird.

## Revendications

1. Procédé de détermination de la viabilité de cellules vivantes, comprenant :
(a) l'obtention d'un récipient chargé de matériaux deutérés ;
(b) l'introduction de cellules vivantes dans le récipient, ce par quoi les cellules vivantes sont en contact avec les matériaux deutérés ;
(c) l'obtention des spectres vibratoires émis par les cellules vivantes ;
dans lequel les spectres vibratoires émis pas les cellules vivantes sont indicatifs du métabolisme, fournissant ainsi une indication de la viabilité des cellules, une plus grande activité métabolique étant indicative d'une plus grande viabilité.

2. Procédé de la revendication 1, dans lequel les spectres vibratoires sont des spectres Raman.

3. Procédé de la revendication 1, dans lequel les spectres vibratoires sont des spectres infrarouge ou des spectres infrarouge proche.

4. Procédé de la revendication 1, dans lequel le matériel deutéré est choisi dans le groupe constitué d'α-D-glucose ; 6,6- dideutéro-α-D-glucose ; D₂O; 3-O-méthylglucose ; 6,6-dideutéro-α-D-3-O-trideutéro-méthylglucose ; 6,6- dideutéro-α-D-3-O-méthylglucose ; et 6,6-dideutéro-α-D-2-O-méthylglucose.

5. Procédé de la revendication 1, dans lequel le matériau deutéré comprend un acide aminé deutéré.

6. Procédé de la revendication 2, comprenant en outre la normalisation des spectres Raman obtenus en comparant les spectres Raman obtenus à un nombre d'onde cible avec les spectres Raman obtenus à un nombre d'onde de référence.

7. Procédé de la revendication 6, dans lequel le nombre d'onde cible est 960 cm⁻¹, 1270 cm⁻¹ ou 2400-2600 cm⁻¹.

8. Procédé de la revendication 6, dans lequel le nombre d'onde de référence est le signal Raman de l'amide I (1600-1700 cm⁻¹).

9. Procédé de la revendication 6, dans lequel le nombre d'onde de référence est le signal Raman de CH₂ (1450 cm⁻¹).

10. Procédé de la revendication 6, dans lequel le nombre d'onde de référence est le signal Raman de la bande CH (2900-3000 cm⁻¹).

11. Procédé de la revendication 6, dans lequel le nombre d'onde de référence est le signal Raman de l'amide III (1200-1350 cm⁻¹).

12. Procédé de la revendication 6, dans lequel le nombre d'onde de référence est l'intégrale de tous les signaux Raman (300-1850 cm⁻¹).

13. Procédé de la revendication 2, comprenant en outre la normalisation des spectres Raman obtenus en comparant les spectres Raman obtenus à un nombre d'onde cible avec la fluorescence générée par une source d'excitation Raman.

14. Procédé de la revendication 13, dans lequel la fluorescence est la fluorescence de H₂O (980 nm).

15. Procédé de la revendication 1, dans lequel le récipient est un tube à centrifuger.

16. Procédé de la revendication 1, comprenant en outre :
(a) l'introduction d'une fraction aliquote des cellules dans un milieu sans matériaux deutérés ;
(b) l'obtention des spectres vibratoires émis par les cellules dans le milieu non deutéré ;
(d) la détermination de la vitesse de décroissance des spectres vibratoires émis ;
dans lequel une vitesse de décroissance plus rapide des spectres vibratoires est indicative d'une viabilité plus grande.

17. Procédé de la revendication 1, dans lequel une cinétique d'échange de D₂O est utilisée comme mesure de la viabilité cellulaire.
